# EUROPEAN PATENT SPECIFICATION

(11) **EP 3 041 941 B1**
(45) Date of publication and mention of the grant of the patent: **27.12.2017**
(21) Application number: 14789632.8
(22) Date of filing: 05.09.2014
(51) Int. Cl.: C12P 1/02

(54) **ENZYME COMPOSITION AND PROCESS OF PREPERATION THEROFF**
ENZYMZUSAMMENSETZUNG UND VERFAHREN ZUR HERSTELLUNG DAVON
COMPOSITION ENZYMATIQUE ET PROCÉDÉ DE PRÉPARATION CORRESPONDANT

(30) Priority: 06.09.2013 IN 2641DE2013
(43) Date of publication of application: 13.07.2016
(73) Proprietor: Council of Scientific and Industrial Research, New Delhi 110001 (IN)
(72) Inventor: KHIRE, Jayant Malhar, Pune 411008 (IN); GUJAR, Pradnya Deepak, Pune 411008 (IN)
(74) Representative: Meissner Bolte Partnerschaft mbB
(86) International application number: PCT/IN2014/000583
(87) International publication number: WO 2015/033356

(56) References cited:
- JP-A- 2007 513 632
- SHAH P ET AL: "Strain improvement and up scaling of phytase production by Aspergillus niger NCIM 563 under submerged fermentation conditions", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 36, no. 3, 10 December 2008 (2008-12-10), pages 373-380, XP019665569, ISSN: 1476-5535
- SONI S K ET AL: "Purification and characterization of two distinct acidic phytases with broad pH stability from Aspergillus niger NCIM 563", WORLD JOURNAL OF MICROBIOLOGY AND BIOTECHNOLOGY, KLUWER ACADEMIC PUBLISHERS, DO, vol. 26, no. 11, 27 March 2010 (2010-03-27), pages 2009-2018, XP019832813, ISSN: 1573-0972

## Description

### FIELD OF INVENTION

The present disclosure relates to an enzyme composition. More specifically, the present disclosure relates to a process for the synthesis of an enzyme composition from *Aspergillus niger* NCIM 563 (MTCC 5213), isolated from soil and its mutant strains *A*. *niger* MCC0013 and MCC0014.

### BACKGROUND OF THE INVENTION

Phytase has low activity under submerged fermentation conditions. The inventors have published that the organism *Aspergillus niger* can produce mutant strains and that both isolate and mutants produce phytase enzyme (Bhavsar et al., Appl Microbiol Biotechnol., 2013, 97(2), 673-9. Hence, the mutants of *Aspergillus niger* NCIM 563 overproducing phytase under such conditions have direct commercial potential.

Mutant strains show higher activity of phytase than earlier reports. New more efficient strains generated using combination of UV and Chemical mutagenesis.

### OBJECTIVE OF THE INVENTION

The main object of the present disclosure is to provide a process for the synthesis of an enzyme composition from *Aspergillus niger* NCIM 563, isolated from soil and its mutant strains *Aspergillus niger* MCC0013 and MCC0014.

### SUMMARY OF THE INVENTION

In an aspect of the present disclosure, there is provided a process for the production of multienzyme system using fermentation, said process comprising: (a) growing *Aspergillus niger* on a medium having sucrose at a concentration of about 1% and yeast extract at a concentration of about 0.2% and at a temperature in the range of 25 - 35 deg C and pH in the range of 5 -6 for a period in the range of 48-120 hours under shaking condition having angular speed in the range of 200 - 250 rpm in order to obtain an inoculum; (b) adding the inoculum, obtained in step (a), to the fermentation medium at the concentration in the range of 10 - 25 percent(v/v) followed by fermenting for a period in the range of 48- 120 hours under shaking condition having angular speed in the range of 200 - 250 rpm for a period in the range of 48-120 hours in order to obtain the fermentation culture; wherein said fermentation medium has the following composition:

| Ingredients | Proportion (percent) |
|---|---|
| starch | 3 - 5 |
| glucose | 0.5- 1.5 |
| carboxy methyl cellulose | 0.5- 1.5 |
| pectin | 0.5 - 1.5 |
| veast extract | 0.6- 1.0 |
| KH₂PO₄ | 0.7- 1.1 |
| NaNO₃ | 1 - 3 |
| MgSO₄.7H₂0 | 0.02-0.04 |
| K₂HPO₄ | 0.2 -0.6 |
| NaCl | 0.4 - 0.8 |
| NH₄Cl | 0.8-1.2 |

and, (c) centrifuging the fermentation culture, obtained in step (b), followed by separation of culture fluid from pellets using filter paper (Whatman no.1) to obtain the said multienzyme system in form of culture fluids.

### DETAILED DESCRIPTION OF THE INVENTION

In an embodiment of the present disclosure, there is provided a process for the production of multienzyme system using fermentation, said process comprising: (a) growing *Aspergillus niger* on a medium having sucrose at a concentration of about 1% and yeast extract at a concentration of about 0.2% and at a temperature in the range of 25 - 35 deg C and pH in the range of 5 -6 for a period in the range of 48-120 hours under shaking condition having angular speed in the range of 200 - 250 rpm in order to obtain an inoculum; (b) adding the inoculum, obtained in step (a), to the fermentation medium at the concentration in the range of 10 - 25 percent(v/v) followed by fermenting for a period in the range of 48- 120 hours under shaking condition having angular speed in the range of 200 - 250 rpm for a period in the range of 48-120 hours in order to obtain the fermentation culture; wherein said fermentation medium has the following composition:

| Ingredients | Proportion (percent) |
|---|---|
| starch | 3 - 5 |
| glucose | 0.5- 1.5 |
| carboxy methyl cellulose | 0.5- 1.5 |
| pectin | 0.5 - 1.5 |
| yeast extract | 0.6-1.0 |
| KH₂PO₄ | 0.7- 1.1 |
| NaNO₂ | 1 - 3 |
| MgSO₄.7H₂0 | 0.02-0.04 |
| K₂HPO₄ | 0.2 -0.6 |
| NaCl | 0.4 - 0.8 |
| NH₄Cl | 0.8-1.2 |

and, (c) centrifuging the fermentation culture, obtained in step (b), followed by separation of culture fluid from pellets using filter paper (Whatman no.1) to obtain the said multienzyme system in form of culture fluids.

In an embodiment of the present disclosure, there is provided an enzyme composition from *Aspergillus niger* NCIM 563 *Aspergillus niger* NCIM 563: NCIM NRS-2 (1953). Parlman strain 3, isolated from soil and its mutant strains *A. niger* MCC0013 and MCC0014.

In an embodiment of the present disclosure, there is provided an enzyme composition comprising CMCase, xylanase, acid phosphatase, amylase, phytase, β-glucosidase, pectinase and α-galactosidase.

In an embodiment of the present disclosure, there is provided an isolated fungal strain *Aspergillus niger* NCIM 563 deposited with Microbial Type Culture Collection and Gene Bank (MTCC) having accession number MTCC 5213.

In an embodiment, the present disclosure, there is provided a process for the synthesis of an enzyme composition comprising CMCase, xylanase, acid phosphatase, amylase, Phytase, β-glucosidase, pectinase and α-galactosidase from *A. nigger* NCIM 563, isolated from soil and its mutant strains *A. niger* MCC0013 and MCC0014, said process being a submerged fermentation process comprising the steps of:
a. Sterilizing the fermentation medium by autoclaving at a temperature in the range of 110 to 140 °C for 15 min;
b. Cooling the fermentation medium of step (a) followed by inoculating the medium with desired spore suspension(1 ml of spore suspension containing 1 x 10⁸ spores/ml) and adjusting the incubation time as per the experimental design;
c. Incubating flasks were at 30 °C at 200 rpm for 4-11 days afforded the desired enzyme composition.

In an embodiment, the present disclosure, there is provided an enzyme composition obtained from *Aspergillus niger* NCIM 563, said composition comprising:
a. CMCase having activity in the range of 0.04 to 0.17 IU/ml;
b. β-glucosidase having activity in the range of 0.52 to 2.04 IU/ml;
c. acid phosphatase having activity in the range of 50.68 to 197.66 IU/ml;
d. Amylase having activity in the range of 1.9 to 7.41 IU/ml;
e. Phytase having activity in the range of 17.53 to 68.30 IU/ml;

In another embodiment of the present disclosure, there is provided an enzyme composition obtained from *Aspergillus niger* mutant NCIM MCC0014, said composition comprising:
a. CMCase having activity in the range of 0.02 to 0.1 IU/ml;
b. Xylanase having activity in the range of 0.05 to 0.19 IU/ml;
c. β-glucosidase having activity in the range of 0.39 to 1.60 IU/ml;
d. Acid phosphatase having activity in the range of 13.08 to 53.36 IU/ml;
e. Amylase having activity in the range of 0.59 to 2.41 IU/ml;
f. Phytase having activity in the range of 38.38 to 156.95 IU/ml;

In yet another embodiment, the present disclosure provides an enzyme composition obtained from *Aspergillus niger* mutant NCIM MCC0014, said composition comprising:
a. CMCase having activity in the range of 0.07 to 0.16 IU/ml;
b. Xylanase having activity in the range of 0.15 to 0.34 IU/ml;
c. β-glucosidase having activity in the range of 0.02 to 0.05 IU/ml;
d. Acid phosphatase having activity in the range of 25.88 to 57.19 IU/ml;
e. Amylase having activity in the range of 1.42 to 3.14 IU/ml;
f. Phytase having activity in the range of 43.22 to 67.51 IU/ml:

### EXAMPLES

The following examples are given by way of illustration of the present invention and therefore should not be construed to limit the scope of the present invention.

### Example 1

### Materials and methods

### Chemicals

Phytic acid sodium salt was purchased from Sigma Chemical Company (St. Louis, MO, USA). All other chemicals used were of analytical grade. Rice bran was purchased from the local market.

### Microorganism, culture media, and enzyme production

*Aspergillus niger* MTCC 5213, used in the present disclosure, was maintained on Potato Dextrose Agar (PDA) slant and stored at 4°C. Spores for inoculation were obtained by culturing the strain at 30°C on a PDA slant for 7 days, followed by washing with 10 ml sterile saline containing 0.01% Tween 80.

The basal fermentation medium according to Bhavsar et al. 2008 contained rice bran, glucose, NaNO₃, MgSO₄.7H₂O, KCl, and FeSO₄.7H₂O. The fermentation medium for optimization via statistical design of experiments included additional components, namely, MnSO₄, dextrin, and Tween 80 at various concentrations as required by the experimental design.

The fermentation medium pH 5.5 before sterilization (100 ml in 250 ml Erlenmeyer flask) was sterilized by autoclaving at 121 °C for 15 min. On cooling the fermentation medium was inoculated with desired spore suspension and the incubation time adjusted as per the experimental design. Flasks were incubated at 30 °C at 200 rpm and samples removed after every 24 h. Enzyme production was expressed as enzyme activity U/ml.

### Partial purification of enzyme

, After fermentation, the mycelium was separated by filtration followed by centrifugation at 10,000×g for 30 min and the clear supernatant was collected. Solid ammonium sulphate was added to the supernatant to 95% saturation with constant stirring. The precipitate was collected by centrifugation at 15,000×g for 20 min and dissolved in minimum volume of 100 mM Glycine-HCl buffer, pH 2.5 and the salt was removed by passing through Sephadex G-25 column. Active fractions were concentrated through YM-30 membrane (Millipore) and used for enzyme activity measurement.

### Example 2

### Production of Different Hydrolytic enzymes

### Parent 563

| | **Day 4** | **Day 7** | **Day 10** | **Day 11** | **Range of Activity (IU/ml)** |
|---|---|---|---|---|---|
| **CMCase (IU/ml)** | 0.04 | 0.08 | 0.13 | 0.17 | 0.04 to 0.17 |
| **Xylanase (IU/ml)** | 0.00 | 0.00 | 0.00 | 0.00 | 0 |
| **β-glucosidase (IU/ml)** | 0.52 | 0.91 | 1.49 | 2.04 | 0.52 to 2.04 |
| **Acid Phosphatase (IU/ml)** | 50.68 | 88.24 | 144.28 | 197.66 | 50.68 to 197.66 |
| **Amylase (IU/ml)** | 1.90 | 3.31 | 5.41 | 7.41 | 1.9 to 7.41 |
| **Phytase (IU/ml)** | 17.53 | 30.52 | 49.99 | 68.30 | 17.53 to 68.30 |

### Mutant MCC0013

| | **Day 4** | **Day 7** | **Day 10** | **Day 11** | **Range of Activity (IU/ml)** |
|---|---|---|---|---|---|
| **CMCase (IU/ml)** | 0.02 | 0.05 | 0.07 | 0.10 | 0.02 to 0.1 |
| **Xylanase (IU/ml)** | 0.05 | 0.10 | 0.14 | 0.19 | 0.05 to 0.19 |
| **β-glucosidase (IU/ml)** | 0.39 | 0.87 | 1.23 | 1.60 | 0.39 to 1.60 |
| **Acid Phosphatase (IU/ml)** | 13.08 | 29.16 | 41.05 | 53.36 | 13.08 to 53.36 |
| **Amylase (IU/ml)** | 0.59 | 1.32 | 1.85 | 2.41 | 0.59 to 2.41 |
| **Phytase (IU/ml)** | 38.38 | 86.00 | 121.21 | 156.95 | 38.38 to 156.95 |

### Mutant MCC0014

| | **Day 4** | **Day 7** | **Day 10** | **Day 11** | **Range of Activity (IU/ml)** |
|---|---|---|---|---|---|
| **CMCase (IU/ml)** | 0.07 | 0.16 | 0.11 | NA | 0.07 to 0.16 |
| **Xylanase (IU/ml)** | 0.15 | 0.34 | 0.24 | NA | 0.15 to 0.34 |
| **β-glucosidase (IU/ml)** | 0.02 | 0.05 | 0.04 | NA | 0.02 to 0.05 |
| **Acid Phosphatase (IU/ml)** | 25.88 | 57.19 | 40.56 | NA | 25.88 to 57.19 |
| **Amylase (IU/ml)** | 1.42 | 3.14 | 2.23 | NA | 1.42 to 3.14 |
| **Phytase (IU/ml)** | 43.22 | 95.46 | 67.51 | NA | 43.22 to 67.51 |

### ADVANTAGES OF THE INVENTION

a. Wider enzyme activities of *Aspergillus niger.*
b. Reduction in fermentation time for mutant NCIM MCC0014 by 7 days and for mutant NCIM MCC0013 by 1 day.
c. Along with increased activity these organisms also show morphological variations under scanning electron microscope. Additionally the activity profile of other enzymes of the mutants and parent culture are different.

## Claims

1. A process for the production of multienzyme system using fermentation, said process comprising:
a) growing *Aspergillus niger* on a medium having sucrose at a concentration of about 1% and yeast extract at a concentration of about 0.2% and at a temperature in the range of 25 - 35 deg C and pH in the range of 5 -6 for a period in the range of 48-120 hours under shaking condition having angular speed in the range of 200 - 250 rpm in order to obtain an inoculum;
b) adding the inoculum, obtained in step (a), to the fermentation medium at the concentration in the range of 10 - 25 percent(v/v) followed by fermenting for a period in the range of 48- 120 hours under shaking condition having angular speed in the range of 200 - 250 rpm for a period in the range of 48-120 hours in order to obtain the fermentation culture; wherein said fermentation medium has the following composition:
| Ingredients | Proportion (percent) |
|---|---|
| starch | 3 - 5 |
| glucose | 0.5- 1.5 |
| carboxy methyl cellulose | 0.5- 1.5 |
| pectin | 0.5 - 1.5 |
| yeast extract | 0.6-1.0 |
| KH₂PO₄ | 0.7- 1.1 |
| NaNO₃ | 1 - 3 |
| MgSO₄.7H₂0 | 0.02-0.04 |
| K₂HPO₄ | 0.2 -0.6 |
| NaCl | 0.4 -0.8 |
| NH₄Cl | 0.8-1.2 |
c) centrifuging the fermentation culture, obtained in step (b), followed by separation of culture fluid from pellets using filter paper (Whatman no.1) to obtain the said multienzyme system in form of culture fluids.

2. The process for the production of multienzyme system as claimed in claim 1 wherein the Aspergillus strain is MTCC 5213.

3. The process for the production of multienzyme system according to claim 1, wherein the multienzyme system comprises the following composition:
| Ingredients | Proportion (units) |
|---|---|
| α-amylase | 6.57-15.47 U |
| amyloglucosidase | 1.11x10⁵-1.21x10⁵ U |
| CMCase | 15.3444.26 U |
| pectinase | 0.04-0.76 U |
| β-galactosidase | 0.34-1.29 U |
| xylanase | 0.41-0.75 U |

## Patentansprüche

1. Verfahren zur Herstellung eines Multienzymsystems unter Verwendung von Fermentation, wobei das Verfahren folgendes umfasst:
a) Züchten von Aspergillus niger auf einem Medium mit Saccharose bei einer Konzentration von etwa 1% und Hefeextrakt bei einer Konzentration von etwa 0,2% und bei einer Temperatur im Bereich von 25 - 35°C und einem pH im Bereich von 5 - 6 für einen Zeitraum in Bereich von 48 - 120 Stunden unter Schüttelbedingungen mit einer Winkelgeschwindigkeit im Bereich von 200-250 U/min, um ein Inokulum zu erhalten;
b) Zugabe des in Schritt (a) erhaltenen Inokulums zu dem Fermentationsmedium bei der Konzentration im Bereich von 10 - 25 % (Vol./Vol.) und anschließende Fermentation für einen Zeitraum in Bereich von 48 - 120 Stunden unter Schüttelbedingungen mit einer Winkelgeschwindigkeit im Bereich von 200 - 250 U/min für einen Zeitraum in Bereich von 48 - 120 Stunden, um die Fermentationskultur zu erhalten; wobei das Fermentationsmedium die folgende Zusammensetzung aufweist:
| Bestandteile | Anteil (%) |
|---|---|
| Stärke | 3-5 |
| Glucose | 0,5 - 1,5 |
| Carboxymethylcellulose | 0,5 - 1,5 |
| Pectin | 0,5 - 1,5 |
| Hefeextrakt | 0,6 - 1.0 |
| KH₂PO₄ | 0,7 - 1,1 |
| NaNO₃ | 1 - 3 |
| MgSO₄.7H₂O | 0,02 - 0,04 |
| K₂HPO₄ | 0,2 - 0,6 |
| NaCl | 0,4 - 0,8 |
| NH₄Cl | 0,8 - 1,2 |
c) Zentrifugieren der in Schritt (b) erhaltenen Fermentationskultur und anschließende Abtrennung des Kulturfluids von Pellets unter Verwendung von Filterpapier (Whatman Nr. 1), um das Multienzymsystem in Form von Kulturfluiden zu erhalten.

2. Verfahren zur Herstellung eines Multienzymsystems nach Anspruch 1, wobei es sich bei dem Aspergillus-Stamm um MTCC 5213 handelt.

3. Verfahren zur Herstellung eines Multienzymsystems nach Anspruch 1, wobei das Multienzymsystem die folgende Zusammensetzung umfasst:
| Bestandteile | Anteil (Units) |
|---|---|
| A-Amylase | 6,75 - 15,47 U |
| Amyloglucosidase | 1,11x10⁵ - 1,21x105 U |
| CMCase | 15,34 - 44,26 U |
| Pectinase | 0,04 - 0,76 U |
| β-Galactosidase | 0,34 - 1,29 U |
| Xylanase | 0,41 - 0,75 U |

## Revendications

1. Procédé pour la production d'un système multi-enzymatique en utilisant la fermentation, dans lequel le procédé comprend les étapes consistant à :
a) cultiver l'Aspergillus niger sur un milieu avec le saccarose à une concentration d'environ 1% et l'extrait de levure à une concentration d'environ 0,2% et à une température dans la plage de 25-35°C et un pH dans la plage de 5-6 pour une période dans la plage de 48-120 heures sous des conditions d'agitation avec une vitesse angulaire dans la plage de 200 - 250°tr/min, afin d'obtenir un inoculum;
b) ajouter l'inoculum obtenu dans l'étape (a) au milieu de fermentation à une concentration dans la plage de 10-25 % (vol./vol.) suivi par la fermentation pendant une période dans la plage de 48-120 heures sous des conditions d'agitation avec une vitesse angulaire dans la plage de 200-250 tr/min pour une période dans la plage de 48-120 heures, afin d'obtenir un inoculum; dans lequel le milieu de fermentation a la composition suivante:
| Ingredients | Pourcentage (%) |
|---|---|
| Amidon | 3-5 |
| Glucose | 0,5 - 1,5 |
| Carboxyméthylcellulose | 0,5 - 1,5 |
| Pectine | 0,5 - 1,5 |
| Extrait de levure | 0,6 - 1,0 |
| KH₂PO₄ | 0,7 - 1,1 |
| NaNO₃ | 1 - 3 |
| MgSO₄.7H₂O | 0,02 - 0,04 |
| K₂HPO₄ | 0,2 - 0,6 |
| NaCl | 0,4 - 0,8 |
| NH₄Cl | 0,8 - 1,2 |
c) centrifuger la culture de fermentation obtenue dans l'étape (b) suivi par la séparation du fluide de culture de pellets en utilisant du papier-filtre (Whatman Nr. 1), afin d'obtenir le système multi-enzymatique en forme de fluides de culture.

2. Procédé pour la production d'un système multi-enzymatique selon la revendication 1, dans lequel la souche d'Aspergillus est MTCC 5213.

3. Procédé pour la production d'un système multi-enzymatique selon la revendication 1, dans lequel le système multi-enzymatique a la composition suivante :
| Ingredients | Quantité d'enzyme (Unités) |
|---|---|
| A-Amylase | 6,75 - 15,47 U |
| Amyloglucosidase | 1,11x10⁵- 1,21x10⁵ U |
| CMCase | 15,34 - 44,26 U |
| Pectinase | 0,04 - 0,76 U |
| β-Galactosidase | 0,34 - 1,29 U |
| Xylanase | 0,41 - 0,75 U |
